(19)

Europäisches Patentamt

European Patent Office

Office européen des brevets

(11) **EP 4 420 523 A1**

(12) **EUROPEAN PATENT APPLICATION**
published in accordance with Art. 153(4) EPC

(43) Date of publication:
**28.08.2024 Bulletin 2024/35**

(21) Application number: **22897755.9**

(22) Date of filing: **21.11.2022**

(51) International Patent Classification (IPC):
*A23J 1/18* (2006.01)    *A23L 33/195* (2016.01)
*A61K 38/01* (2006.01)    *A61P 3/02* (2006.01)

(86) International application number:
**PCT/CN2022/133213**

(87) International publication number:
**WO 2023/093672 (01.06.2023 Gazette 2023/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.11.2021 CN 202111418178**

(71) Applicants:
• **Angel Yeast Co., Ltd**
**Yichang, Hubei 443003 (CN)**
• **Angel Nutritech Co., Ltd**
**Yichang, Hubei 443003 (CN)**

(72) Inventors:
• **CHEN, Zhixian**
**Yichang, Hubei 443003 (CN)**

• **XIONG, Tao**
**Yichang, Hubei 443003 (CN)**
• **ZHU, Yinhong**
**Yichang, Hubei 443003 (CN)**
• **ZHANG, Haibo**
**Yichang, Hubei 443003 (CN)**
• **GONG, Shiyu**
**Yichang, Hubei 443003 (CN)**
• **DONG, Yunhai**
**Yichang, Hubei 443003 (CN)**

(74) Representative: **karo IP**
**karo IP Patentanwälte**
**Kahlhöfer Rößler Kreuels PartG mbB**
**Postfach 32 01 02**
**40416 Düsseldorf (DE)**

(54) **YEAST PROTEIN, COMPOSITION THEREOF, PREPARATION METHOD THEREFOR, AND USE OF YEAST PROTEIN AND COMPOSITION**

(57)    The present invention relates to the technical field of medicine, and specifically relates to a yeast protein, a composition thereof, a preparation method therefor, and the use of the yeast protein and the composition. The outer layer of the yeast protein of the present invention is coated with zymosan; the yeast protein is in the shape of a sphere or an ellipsoid; the yeast protein has a protein content of 70% or more and a zymosan content of 5%-30%, calculated as the weight percentage of a dry base. The yeast protein of the present invention has obviously lower digestibility than that of soybean protein isolate and whey protein in the stomach, causes a stronger satiety after being eaten compared with whey protein, has the characteristic of slow digestion, and is a slow-digesting protein. The yeast protein of the present invention also contains 18 amino acids, including eight essential amino acids and two semi-essential amino acids for the human body, and thus is a complete protein due to the full type of amino acids. The yeast protein can be used as the slow-digesting protein in the preparation of a composition which is intended to be enterally administered to mammals so as to regulate the postprandial amino acid level in the blood plasma.

FIG. 7

EP 4 420 523 A1

## Description

**Technical Field**

The present invention relates to the technical field of medicine and particularly relates to a yeast protein, a composition thereof, a preparation method therefor, and the use of the yeast protein and the composition.

**Background Art**

Proteins are an indispensable class of substances in life activities, which constitute the basic organic matter of cells. The human body must maintain the body's protein renewal through constant protein intake. After being digested and hydrolyzed into amino acids in the body, the ingested protein is synthesized into proteins needed by the human body, at the same time, the new proteins are constantly metabolized and decomposed, and are in dynamic equilibrium all the time. With the development of modern society, the demand for human life has become more and more diversified. In order to pursue a healthier lifestyle and to meet people of different occupations, different ages, and different needs, a food product that maintains the protein balance of the body for a long time is becoming the focus of attention. As this kind of protein, slowly digested protein can increase the postprandial protein acquisition, reduce the metabolic overload of some organs or some enzymes, limit daily food intake, improve tissue regeneration, and make up for some functional disorders in amino acid metabolism. It is urgent to find a protein with a slow digestion function.

Chinese patent CN107802001A provides a preparation method for a protein peptide - slow-digesting starch granule, wherein a protein peptide - slow-digesting starch complex is obtained by mixing rice starch, a protein peptide, and water and then homogenizing the same, and the protein peptide - slow-digesting starch granule is obtained by spray drying the complex. European patent WO1997005785A1 describes a composition for neonatal food products containing slow-digesting proteins, including naturally slower-digestible casein, which has been previously denatured in order to slow down the digestion rate.

Yeast protein is a high-quality complete protein present in natural yeast. Yeast proteins contain a complete population of amino acids, including the eight amino acids essential to the human body, particularly lysine, which is present in lower amounts in cereal proteins and higher amounts in yeast. In addition, the proportion of amino acids in yeast is close to the ideal amino acid composition value recommended by the Food and Agriculture Organization of the United Nations (FAO), so its nutritional value is higher. Currently, yeast proteins are mainly used in the field of food products and feed.

**Summary of the Invention**

The technical problem to be solved by the present invention is that the prior art lacks a slow-digesting yeast protein.

In view of the deficiencies of the prior art, the first object of the present invention to provide a yeast protein, the outer layer of which is coated with zymosan in the shape of a sphere or an ellipsoid, based on taking a dry base weight of the yeast protein as 100%, the protein content is more than 70%, the zymosan content is 5-30%, and the gastric digestibility is significantly lower than that of soybean protein isolate and whey protein; the second object of the present invention is to provide a preparation method for the above-mentioned yeast; the third object of the present invention is to provide a composition of the above-mentioned yeast; the fourth object of the present invention is to provide the use of the above-described yeast proteins and compositions thereof as slow-digesting proteins, which can be used for the preparation of compositions intended for enteral administration to mammals for the modulation of postprandial amino acid level in the blood plasma.

Technical solution of the present invention:

The present invention provides a yeast protein, the outer layer of the yeast protein is coated with zymosan, the yeast protein is in the shape of a sphere or an ellipsoid, and based on taking a dry base weight of the yeast protein as 100%, the yeast protein has a protein content of 70% or more and a zymosan content of 5-30%.

Preferably, the protein comprises isoleucine, leucine, lysine, methionine, phenylalanine, tyrosine, threonine, cysteine, tryptophan, valine, aspartic acid, arginine, glutamic acid, glycine, histidine, alanine, serine, and proline.

Preferably, by weight of the yeast protein, the content of isoleucine is 60 mg/g or more, the content of leucine is 90 mg/g or more, the content of lysine is 96 mg/g or more, the content of methionine is 25 mg/g or more, the content of phenylalanine is 49 mg/g or more, the content of tyrosine is 49 mg/g or more, the content of threonine is 50 mg/g or more, the content of cysteine is 14 mg/g or more, the content of tryptophan is 13 mg/g or more, the content of valine is 55 mg/g or more, the content of aspartic acid is 111 mg/g or more, the content of arginine is 53 mg/g or more, the content of glutamic acid is 109 mg/g or more, the content of glycine is 47 mg/g or more, the content of histidine is 30 mg/g or more, the content of alanine is 59 mg/g or more, the content of serine is 52 mg/g or more, and the content of proline is 27 mg/g or more.

The present invention also provides a preparation method for the above-mentioned yeast protein, comprising the following steps:

(1) adding an enzyme to the yeast for enzymolysis;
(2) performing thermal extraction on the enzymatic hydrolysate obtained in step (1), and centrifuging to take a heavy phase to obtain the yeast protein.

Preferably, in the above preparation method, the enzyme in step (1) is selected from α-mannanase and/or β-glucanase; preferably, the enzyme is added in an amount of 0.05-0.5 wt%, preferably 0.1-0.3 wt% of the yeast content; further preferably, the enzymolysis temperature is in the range of 40-70°C, preferably 50-60°C, still further preferably, the enzymolysis time is in the range of 8-24 h, preferably 14-18 h.

Preferably, in the above preparation method, in step (1), the yeast is formulated into a solution with a mass concentration of 5%-15%, preferably 8-12%, and preferably the yeast is beer yeast or Saccharomyces cerevisiae.

Preferably, in the above preparation method, the thermal extraction pH in step (2) is 7.0-8.0, preferably 7.5-7.7, preferably the thermal extraction temperature is 70-90°C, preferably 75-80°C, further preferably the thermal extraction time is 1-2 h.

Preferably, in the above preparation method, further comprising subjecting the yeast protein to a drying step: preparing the yeast protein into a dispersion with a mass concentration of 5-20%, and then drying to obtain, preferably the dispersion has a mass concentration of 10-15%.

The present invention also provides a yeast protein obtained by the above preparation method.

The present invention also provides a yeast protein composition comprising at least the yeast protein described above.

Preferably, the yeast protein composition further comprises one or a combination of two or more selected from the group consisting of carbohydrates, lipid substances, and free amino acids; preferably, the carbohydrate is one or a combination of two or more selected from the group consisting of corn syrup, maltodextrin, sucrose, oat powder, corn powder, red date flour, red bean flour, Radix Puerariae flour, pumpkin flour, chia seed, and lactose; further preferably, the lipid substance is one or a combination of two or more selected from the group consisting of coconut oil, Brassica oil, corn oil, and soybean lecithin; furthermore preferably, the free amino acid is one or a combination of two or more selected from the group consisting of arginine, L-cystine, L-glutamine, L-glycine, L-histidine, L-isoleucine, L-leucine, L-lysine, L-methionine, L-proline, L-tryptophan, L-tyrosine and L-valine.

The present invention also provides the use of a yeast protein as described above or a yeast protein composition as described above for the preparation of a composition intended for enteral administration to a mammal for the modulation of postprandial amino acid level in the blood plasma.

Preferably, the yeast protein or yeast protein composition is used for the preparation of a composition for increasing postprandial protein acquisition.

Preferably, the yeast protein or yeast protein composition is used for the preparation of a composition for reducing the metabolic overload of certain organs and/or certain enzymes. Preferably, the yeast protein or yeast protein composition is used for the preparation of a composition for limiting daily food intake.

Preferably, the yeast protein or yeast protein composition is used for the preparation of a composition for compensating certain dysfunctions in amino acid metabolism.

Preferably, the yeast protein or yeast protein composition is used for the preparation of a composition for improving tissue regeneration.

The present invention also provides a food product for patients with renal dysfunction comprising, in percentage by weight, the following components: the yeast protein 1-10%, carbohydrates 20-30%, and lipid compounds 5-15%, the remainder being water.

The present invention also provides a meal replacement powder comprising, in parts by weight, the following components: 5-20 parts of yeast protein, 5-20 parts of oat powder, 5-25 parts of corn powder, 5-20 parts of red date powder, 5-10 parts of red bean powder, 5-10 parts of Radix Puerariae powder, 5-10 parts of pumpkin powder, and 10-30 parts of chia seed.

The present invention also provides a food product for a patient with phenylketonuria comprising, in percentage by weight, the following components: the yeast protein 1-5%, free amino acids 1-5%, carbohydrates 10-20% and lipid compounds 1-5%, the remainder being water.

Advantageous effects of the present invention:

In the present invention, the process of thermal extraction after enzymolysis is used, so that the yeast protein still has the structure of yeast cells, a layer of zymosan is coated on the outside of the protein, and the presence of the zymosan may hinder the attack site of the digestive enzyme in vivo, thus slowing down the digestion of the protein; meanwhile, thermal extraction can remove nucleic acid from yeast and reduce the content of nucleic acid in yeast protein.

The outer layer of the yeast protein of the present invention is coated with zymosan, the yeast protein is in the shape of a sphere or an ellipsoid, the yeast protein has a protein content of 70% or more and a zymosan content of 5-30%; the yeast protein has obviously lower digestibility than that of soybean protein isolate and whey protein in the stomach, causes a stronger satiety after being eaten compared with whey protein, has the characteristic of slow digestion, and is a slow-digesting protein. The yeast protein of the present invention also contains 18 amino acids, including eight essential amino acids and two semi-essential amino acids for the human body, and thus is a complete protein due to the full type of amino acids. The yeast protein can be used as the slow-digesting protein in the preparation of a composition which is intended to be enterally administered to mammals so as to regulate the postprandial amino acid level in the blood plasma.

## Brief Description of the Drawings

FIG. 1 is a graph showing the percentage of digestion or absorption of the yeast protein obtained in Example 1, the yeast protein produced in Comparative Example 1, the soybean protein isolate,
and the whey protein at different inspection time points;
FIG. 2 is a graph of the perceived hunger curve for the satiety test of Experimental Example 2;
FIG. 3 is a hunger curve of the satiety test of Experimental Example 2;
FIG. 4 is a satiety curve of the satiety test of Experimental Example 2;
FIG. 5 is a scanning electron microscope image of the yeast protein obtained in Example 1 at a magnification of 1000 times;
FIG. 6 is a scanning electron microscope image of the yeast protein obtained in Example 1 at a magnification of 2000 times;
FIG. 7 is a scanning electron microscope image of the yeast protein obtained in Example 1 at a magnification of 10000 times;
FIG. 8 is a scanning electron microscope image of the yeast protein obtained in Comparative Example 1 at a magnification of 3000 times;
FIG. 9 is a scanning electron microscope image of the soy protein isolate at a magnification of 300 times;
FIG. 10 is a scanning electron microscope image of the raw Saccharomyces cerevisiae powder of Example 1 at a magnification of 2000 times.

The marks in the figures are illustrated as follows: YPC-the yeast protein prepared in Example 1, YPT-the yeast protein prepared in Comparative Example 1, WPC-whey protein, and SPI-soybean protein isolate.

## Detailed Description of the Invention

In order that the objects, aspects, and advantages of the present invention will become more apparent, a detailed description of the aspects of the present invention will be provided below. It is to be understood that the described embodiments are only a few, but not all embodiments of the present invention. Based on the examples of the present invention, all other embodiments obtained by a person of ordinary skill in the art without inventive effort fall within the scope of the present invention.

The present invention provides a yeast protein, the outer layer of the yeast protein is coated with zymosan, the yeast protein is in the shape of a sphere or an ellipsoid, and based on taking a dry base weight of the yeast protein as 100%, the yeast protein has a protein content of 70% or more and a zymosan content of 5-30%. The yeast proteins of the present invention still have the structure of a yeast cell, and the protein is coated with a layer of zymosan, and the presence of the zymosan hinders the site of attack by digestive enzymes in vivo so that the digestion of the protein is slowed. The gastric digestibility of the yeast protein was significantly lower than that of soybean protein isolate and whey protein, and the satiety after being eaten was stronger than that of whey protein. The yeast protein was a slow-digesting protein.

Wherein, the protein contains isoleucine (Ile), leucine (Leu), lysine (Lys), methionine (Met), phenylalanine (Phe), tyrosine (Tyr), threonine (Thr), cysteine (Cys), tryptophan (Trp), valine (Val), aspartic acid (Asp), arginine (Arg), glutamic acid (Glu), glycine (Gly), histidine (His), alanine (Ala), Serine (Ser) and proline (Pro). Preferably, by weight of the yeast protein, the content of isoleucine is 60 mg/g or more, the content of leucine is 90 mg/g or more, the content of lysine is 96 mg/g or more, the content of methionine is 25 mg/g or more, the content of phenylalanine is 49 mg/g or more, the content of tyrosine is 49 mg/g or more, the content of threonine is 50 mg/g or more, the content of cysteine is 14 mg/g or more, the content of tryptophan is 13 mg/g or more, the content of valine is 55 mg/g or more, the content of aspartic acid is 111 mg/g or more, the content of arginine is 53 mg/g or more, the content of glutamic acid is 109 mg/g or more, the content of glycine is 47 mg/g or more, the content of histidine is 30 mg/g or more, the content of alanine is 59 mg/g or more, the content of serine is 52 mg/g or more, and the content of proline is 27 mg/g or more.

The present invention also provides a preparation method for the above-mentioned yeast protein, comprising the following steps:

(1) adding an enzyme to the yeast for enzymolysis;
(2) performing thermal extraction on the enzymatic hydrolysate obtained in step (1), and centrifuging to take a heavy phase to obtain the yeast protein.

Wherein, the enzyme in step (1) is selected from α-mannanase and/or β-glucanase; the enzyme is added in an amount of 0.05-0.5 wt%, preferably 0.1-0.3 wt% of the yeast content; the enzymolysis temperature is 40-70°C, preferably 50-60°C; the enzymolysis time is 8-24 h, preferably 14-18 h. Step (1), before the yeast enzymolysis, further comprising formulating the yeast into a solution with a mass concentration of 5%-15%, preferably 8-12%; the yeast is beer yeast or Saccharomyces cerevisiae.

the thermal extraction pH of step (2) is 7.0-8.0, preferably 7.5-7.7; preferably, the thermal extraction temperature is from 70-90°C, preferably 75-80°C; further preferably, the thermal extraction time is 1-2 h.

The above-mentioned yeast protein preparation method further comprises subjecting the yeast protein to a drying step: preparing the yeast protein into a dispersion with a mass concentration of 5-20%, and then drying to obtain, preferably the dispersion has a mass concentration of 10-15%. The present invention also provides a yeast protein prepared by the above-described preparation method.

The present invention also provides a yeast protein composition comprising at least the yeast protein described above.

Wherein, the yeast protein composition further comprises one or a combination of two or more selected from the group consisting of carbohydrates, lipid substances, and free amino acids; the carbohydrate is one or a combination of two or more selected from the group consisting of corn syrup, maltodextrin, sucrose, oat powder, corn powder, red date powder, red bean powder, Radix Puerariae powder, pumpkin powder, chia seed, and lactose; the lipid substance is one or a combination of two or more selected from the group consisting of coconut oil, Brassica oil, corn oil, and soybean lecithin; the free amino acid is one or a combination of two or more selected from the group consisting of arginine, L-cystine, L-glutamine, L-glycine, L-histidine, L-isoleucine, L-leucine, L-lysine, L-methionine, L-proline, L-tryptophan, L-tyrosine, and L-valine.

The present invention further provides the use of a yeast protein or yeast protein composition as described above for the preparation of a composition intended for enteral administration to a mammal for the modulation of postprandial amino acid level in the blood plasma. To

1) increase postprandial protein acquisition, and/or
2) avoid metabolic overload of certain organs and/or certain enzymes, and/or
3) limit daily food intake by virtue of the satiating effect of these proteins, and/or
4) make up for certain dysfunctions in amino acid metabolism, more for enzyme deficiencies, and/or
5) increase tissue regeneration.

The yeast protein of the present invention, as a slow-digesting protein, may avoid metabolic overload of certain organs and/or certain enzymes. After eating a diet containing various nitrogenous compounds (proteins, peptides, amino acids), the liver tries to keep the amino acid concentration within physiological limits by breaking down part of the amino acids from the diet. The moderate attainment of dietary amino acids can reduce the overactivity of the organ presenting the disorder and thus make it possible to avoid overwork. Patients with renal abnormalities require strict control of protein intake, often receiving a low protein diet, to reduce the production of nitrogenous waste products, while the intake of slow-digesting proteins has the effect of reducing the production of nitrogen that should be subsequently eliminated by the kidney, distributing this production over a longer period of time and increasing the satiety of this protein in order to ensure better tolerance of this diet.

Ingestion of the yeast proteins of the present invention can be beneficial to improve the digestive process for certain people who are deficient in digestive enzymes, such as when they are deficient in trypsin. This benefit is ensured by reducing the amount of substrate to be hydrolyzed by the proteolytic enzyme from the pancreas thereby obtaining a better enzyme/substrate ratio.

Accumulation of these amino acids or one of their degradation products will produce neurological and clinical syndromes in diseases where there is dysfunction in amino acid metabolism and more particularly where there is an enzyme deficiency in these amino acid degradation pathways (e.g. phenylalaninemia and phenylketonuria, hypertyrosinemia, histidinemia, homocystinuria, amino acid diseases associated with branched-chain amino acids). To avoid this accumulation, dietary therapy is prescribed. It consists of a diet containing no- or very little-amino acids involved in causing the disease. Specific products developed for these populations contain either free amino acids or highly hydrolyzed proteins. However, these mixtures do not have a pleasant taste. Furthermore, in order to avoid diarrhoea caused by the high permeability of the product, the consumer should ingest the product in the form of a small meal. The yeast protein of the present invention has both a slow-digesting rate and a small amount of proteins involving amino acids making it possible to improve the taste of the diet and thus increase its tolerance, thereby limiting the risk of diarrhoea, avoiding amino acid fluctuations in the plasma and increasing the postprandial protein acquisition.

The yeast protein of the present invention, as a slow-digesting protein, is also conceivable for use in the elderly, in which the amount of body protein is reduced and the effects on autonomy, resistance to aggression (disease, different stress) and the ability to recover after aggression are reduced as compared to young subjects, and the slow-digesting protein can continuously provide a supply of protein to the body through slow digestion. Moreover, aging is accompanied by a decrease in kidney viability. The slow-digesting protein thus makes it possible to avoid renal overload by better preserving the amount of protein.

The present invention also provides a food product for patients with renal dysfunction comprising, in percentage by weight: the yeast protein 1-10%, carbohydrates 20-30%, and lipid compounds 5-15%, the remainder being water.

The present invention also provides a meal replacement powder comprising, in parts by weight, the following components: 5-20 parts of yeast protein, 5-20 parts of oat powder, 5-25 parts of corn powder, 5-20 parts of red date powder, 5-10 parts of red bean powder, 5-10 parts of Radix Puerariae powder, 5-10 parts of pumpkin powder, and 10-30 parts of chia seed.

The present invention also provides a food product for a patient with phenylketonuria comprising, in percentage by weight, the yeast protein 1-5%, free amino acids 1-5%, carbohydrates 10-20%, and lipid compounds 1-5%, the remainder being water.

Advantageous effects of the present invention will be further illustrated by specific examples and test examples.

The reagents and instrumental information used in the examples of the present invention are shown in Table 1:

Table 1 Reagents and instruments information

| Reagents | Purity or Model | Vendors |
|---|---|---|
| β-glucanase | Enzyme activity 300 U | Angel Yeast Co., Ltd. |
| α-mannanase | Enzyme activity 30,000 U | DOING-HIGHER BIO-TECH Co., Ltd. |
| Saccharomyces cerevisiae powder | F55, 97% | Angel Yeast Co., Ltd. |
| Corn syrup solids | 99% | Jinan Shenghe Chemical Co., Ltd. |
| Maltodextrin | 99% | Shandong Xiwang Food Co., Ltd. |
| Sucrose | 99% | Sichuan Litian Industrial Co., Ltd. |
| Coconut oil | 99% | Zhengzhou Shiquanshimei Commercial and Trading Co., Ltd. |
| Brassica oil | 99% | Zhengzhou Shiquanshimei Commercial and Trading Co., Ltd. |
| Corn oil | 99% | Shandong Xiwang Food Co., Ltd. |
| Soybean lecithin | 98% | Xi'an Zhanxun Biotechnology Co., Ltd. |
| Oat powder | 98% | Xinghua Fenghe Food Co., Ltd. |
| Corn powder | 98% | Xinghua Fenghe Food Co., Ltd. |
| Red date powder | 98% | Xinghua Fenghe Food Co., Ltd. |
| Red bean powder | 98% | Xinghua Fenghe Food Co., Ltd. |
| Pumpkin powder | 98% | Xinghua Fenghe Food Co., Ltd. |
| Chia seed | 99% | Shaanxi Bovlin Biotechnology Co., Ltd. |
| Lactose | 99% | Shandong Jurong Bioengineering Co., Ltd. |
| Soy protein isolate | 90% | Yihai Kerry Arawana Holdings Co., Ltd. |

(continued)

| Reagents | Purity or Model | Vendors |
|---|---|---|
| Whey protein | 80% | Clanbia Group |
| Arginine | 99% | Hebei Lihua Biotechnology Co., Ltd. |
| L-Cystine | 99% | Hebei Lihua Biotechnology Co., Ltd. |
| L-Glutamine | 99% | Hebei Lihua Biotechnology Co., Ltd. |
| L-Glycine | 99% | Hebei Lihua Biotechnology Co., Ltd. |
| L-Histidine | 99% | Hebei Lihua Biotechnology Co., Ltd. |
| L-isoleucine | 99% | Hebei Lihua Biotechnology Co., Ltd. |
| L-leucine | 99% | Hebei Lihua Biotechnology Co., Ltd. |
| L-Lysine | 99% | Hebei Lihua Biotechnology Co., Ltd. |
| L-Methionine | 99% | Hebei Lihua Biotechnology Co., Ltd. |
| L-proline | 99% | Hebei Lihua Biotechnology Co., Ltd. |
| L-tryptophan | 99% | Hebei Lihua Biotechnology Co., Ltd. |
| L-tyrosine | 99% | Hebei Lihua Biotechnology Co., Ltd. |
| L-valine | 99% | Hebei Lihua Biotechnology Co., Ltd. |
| Fat-soluble vitamin | | NHU Co., Ltd. |
| Water-soluble vitamin | | NHU Co., Ltd. |

**Example 1**

10 kg of Saccharomyces cerevisiae powder was added into purified water to prepare a solution of 200 kg, dilute hydrochloric acid was added to adjust pH to 5.0, the temperature was adjusted to 40°C, and 5 g of α-mannanase was added for enzymolysis for 24 h; the pH was adjusted to 7.0 with sodium hydroxide, the temperature was raised to 70°C, and the temperature was maintained for 1 h; after cooling to 50°C and centrifuging at 5,000 rpm for 20 min, the upper layer liquid was removed, then the lower layer solid was prepared into a dispersion liquid with a mass concentration of 5% and a yeast protein product was obtained by spray drying.

The contents of proteins in the yeast proteins prepared in Examples were determined by the Kjeldahl method. The results are shown in Table 2.

The prepared yeast protein was then assayed for moisture, zymosan, and nucleic acid content as follows. The assay results are shown in Table 2.

1. Method for determining zymosan

The content of zymosan was determined by liquid chromatography:

(1) Sample processing: 400 mg (accurate to 0.1 mg) of the sample was accurately weighed and placed into a small test tube with a screw cap made of 20 mL heat-resistant glass, 6.0 mL hydrochloric acid (37%) was added, the vial cap was carefully tightened and then a vortex mixer was used to mix, so as to obtain a uniform suspension. The vials were placed in a 30°C water bath to process for 45 min and vortex mixed once every 15 min. The suspension was then quantitatively transferred to a 200 mL Dewar flask, and the small test tube was washed with about 100 mL to 120 mL of water in several portions, and the washing solution was incorporated into the Dewar flask. The Dewar flask was placed in an autoclave and processed for 60 min at 121°C. After removal and cooling, the solution was adjusted to pH 6-7 with sodium hydroxide solution and then brought to a volume to 200 mL. Cellulose acetate membranes with a pore size of 0.45 microns were used for filtration before use. At the same time, 200 mg of glucanase control sample was accurately weighed, and the same treatment was performed according to the sample treatment method.

(2) Chromatographic conditions: pure water was used as the mobile phase, the flow rate was 0.5 mL/min, the column temperature was 80°C, and the sample was injected after the baseline of the instrument was stable.

(3) Drawing of a standard curve: 1, 2, 3, 4, and 5 mL to 10 mL of mannose and glucose standard solution were respectively sucked into a volumetric flask, high purity water was used to bring volume to scale, so as to obtain mixed standard samples with mannose and glucose of 200, 400, 600, 800 and 1000 mg/L respectively. 20 uL sample was accurately injected under the above chromatographic conditions, to obtain the regression equation between the chromatographic peak area and the concentration of the standard substance, and the standard curve was drawn.

(4) Determination of samples and control samples: under the same chromatographic conditions, the treated sample, mannose, and glucanase control sample were injected into the chromatograph respectively, and record the retention time and peak area of each chromatographic peak. Qualify with the retention time of the chromatographic peak of sugar standard and quantify with the peak area of the chromatographic peak of sugar standard.

(5) The content of glucan or manno oligosaccharide is calculated according to the following formula:

$$X = \frac{A_1 \times 0.2 \times 100}{m_1 \times 1000} \times 0.9 \times F \quad\quad\quad (1)$$

$$F = \frac{P \times (100 - W)}{\dfrac{A_2 \times 0.2 \times 100}{m_2 \times 1000} \times 0.9} \quad\quad\quad (2)$$

Content of zymosan -content of glucan + content of manno oligosaccharide

In the formula:

X---Content of glucan or manno oligosaccharide in the sample, %;

$A_1$---Content of glucose or mannose in the sample solution searched on the standard curve according to the peak area of sample solution, mg/L;

$A_2$---Content of glucose in the sample solution searched on the standard curve according to the peak area of glucanase control sample solution, mg/L;

m1---Mass of sample weighed, g;

m2---Mass of the glucanase control sample, g;

0.2 --- Volume of volume making up after treatment of sample or glucanase control sample, L;

0.9---Coefficient for converting glucose or mannose into glucanase or manno oligosaccharide;

F---Empirical compensation factor for the low result caused by the destruction of glucose and mannose in the acid hydrolysis of the sample;

P---Purity of glucanase control sample (according to the test report provided by the reagent manufacturer);

W-Moisture of glucanase control sample (according to the test report provided by the reagent manufacturer).

2. Nucleic acid content determination method

Determine total phosphorus according to GB/T 6437 *Determination of phosphorus in feed-Spectphotometry,* and calculate according to the following formula: nucleic acid content = total phosphorus x 340/32. The specific operations are: phosphorus in the nucleic acid of yeast hydrolysate was digested to produce inorganic phosphorus which was dissolved in water, and reacted with ammonium molybdate to produce ammonium phosphomolybdate. Under acidic conditions, molybdenum blue was produced with a reducing agent. The color of molybdenum blue was proportional to the content of $P_2O_5$, which was consistent with Lambert-Beer law. The absorbance at 700 nm was measured with a spectrophotometer.

3. Moisture determination method

According to the method specified in 6.2 of the national standard GB/T 23530-2009, a certain mass of sample was taken, dried at 103°C for 4 h to constant weight, and weighed to calculate the moisture content.

**Example 2**

30 kg of Saccharomyces cerevisiae powder was added into purified water to prepare a solution of 200 kg, dilute hydrochloric acid was added to adjust pH to 5.0, the temperature was adjusted to 50°C, and 30g of β-glucanase was added for enzymolysis for 18 h; the pH was adjusted to 7.5 with sodium hydroxide, the temperature was raised to 80°C, and the temperature was maintained for 2 h; after cooling to 50°C and centrifuging at 5,000 rpm for 20 min, the upper layer liquid was removed, then the lower layer solid was prepared into a dispersion liquid with a mass concentration of 10%, and a yeast protein product was obtained by spray drying.

The protein content, zymosan content, moisture, and nucleic acid content of the prepared yeast protein were measured in the same manner as in Example 1, and the results were shown in Table 2.

**Example 3**

16kg of Saccharomyces cerevisiae powder was added into purified water to prepare a solution of 200 kg, dilute hydrochloric acid was added to adjust pH to 5.0, the temperature was adjusted to 60°C, and 24 g of $\alpha$-mannanase and 24 g of $\beta$-glucanase were added for enzymolysis for 14 h; the pH was adjusted to 7.7 with sodium hydroxide, the temperature was raised to 75°C, and the temperature was maintained for 2 h; after cooling to 50°C and centrifuging at 5000 rpm for 20 min, the upper layer liquid was removed, then the lower layer solid was prepared into a dispersion liquid with a mass concentration of 15%, and a yeast protein product was obtained by spray drying.

The protein content, zymosan content, moisture, and nucleic acid content of the prepared yeast protein were measured in the same manner as in Example 1, and the results were shown in Table 2.

**Example 4**

24 kg of Saccharomyces cerevisiae powder was added into purified water to prepare a solution of 200 kg, dilute hydrochloric acid was added to adjust pH to 5.0, the temperature was adjusted to 70°C, and 120g of $\alpha$-mannanase was added for enzymolysis for 8 h; the pH was adjusted to 7.7 with sodium hydroxide, the temperature was raised to 90°C, and the temperature was maintained for 1.5 h; after cooling to 50°C and centrifuging at 5000 rpm for 20 min, the upper layer liquid was removed, then the lower layer solid was prepared into a dispersion liquid with a mass concentration of 20%, and a yeast protein product was obtained by spray drying.

The protein content, zymosan content, moisture, and nucleic acid content of the prepared yeast protein were measured in the same manner as in Example 1, and the results were shown in Table 2.

**Example 5**

Water was purified by reverse osmosis membrane, 1 L of water was taken and heated to 70°C, 50 g of yeast protein prepared in Example 1, 151 g of corn syrup solids, 93 g of maltodextrin, and 26 g of sucrose were added, then 40 g of coconut oil, 24 g of Brassica oil, 11 g of corn oil and 4.8 g of soybean lecithin were added, the mixture was homogenized at 150°C by injecting steam, cooled to 75°C, 0.8 g of minerals and 1.5 g of water-soluble vitamins were added, and then aseptically packaged in a container to obtain a beverage for patients with renal dysfunction having an energy density of 200 Kcal/100mL.

**Example 6**

Formulation composition: 20 g of the yeast protein prepared in Example 1, 5 g of oat powder, 10 g of corn powder, 15 g of red date powder, 5 g of red bean powder, 5 g of Radix Puerariae powder, 10 g of pumpkin powder and 15 g of chia seed;

the above formula composition is directly packaged after sieving to obtain meal replacement powder.

**Example 7**

Water was purified by reverse osmosis membrane, 1 L of water was heated to 70°C, 17 g of yeast protein prepared in Example 1, 1 g of arginine, 1 g of L-cystine, 1 g of L-glutamine, 1 g of L-glycine, 1 g of L-histidine, 1.5 g of L-isoleucine, 2 g of L-leucine, 2 g of L-lysine, 1 g of L-methionine, 1 g of L-proline, 1 g of L-tryptophan, 2 g of L-tyrosine and 0.5 g of L-valine were added, 130 g of lactose was added, followed by 24 g of Brassica oil, 16 g of corn oil, 0.5g soybean lecithin and 0.5 g fat-soluble vitamins, the mixture was heated by injecting steam at 80°C for 5 min, cooled to 60°C, 0.8 g minerals and 1.5 g water-soluble vitamins were added, the mixture was homogenized in 2 stages, which was first homogenized at 10 mPa and then at 7 mPa and finally spray dried to a food product for a patient with phenylketonuria with a water content of 4%.

**Comparative Example 1**

The yeast protein was prepared according to the method in patent CN109198156. 10 kg of Saccharomyces cerevisiae powder was added into purified water to prepare a solution of 200 kg, dilute hydrochloric acid was added to adjust pH to 5.0, the temperature was adjusted to 40°C, and 5 g of $\alpha$-mannanase was added for enzymolysis for 24 h; after centrifugation at 5,000 rpm for 20 min, the supernatant was removed, the precipitate was dispersed with 60 kg of water, the pH was adjusted to 7.0 with sodium hydroxide, the above liquid was homogenized at 800 bar pressure for 6 times at a flow rate of 1 m$^3$/h, and the crude protein was freeze-dried to obtain a yeast protein product.

Table 2 Component content determination results of different yeast proteins

|  | Protein content, % | zymosan content, % | Moisture, % | Nucleic acid content, % |
|---|---|---|---|---|
| Example 1 | 70.6 | 21.8 | 3.55 | 0.2 |
| Example 2 | 73.3 | 19.6 | 3.02 | 0.3 |
| Example 3 | 81.6 | 7.3 | 3.42 | 0.1 |
| Example 4 | 77.1 | 16.5 | 3.53 | 0.1 |
| Comparative Example 1 | 70.7 | 23.1 | 2.98 | 0.3 |

The yeast proteins prepared in Example 1 and Comparative Example 1 above were characterized by:

1. Scanning electron microscope SEM images

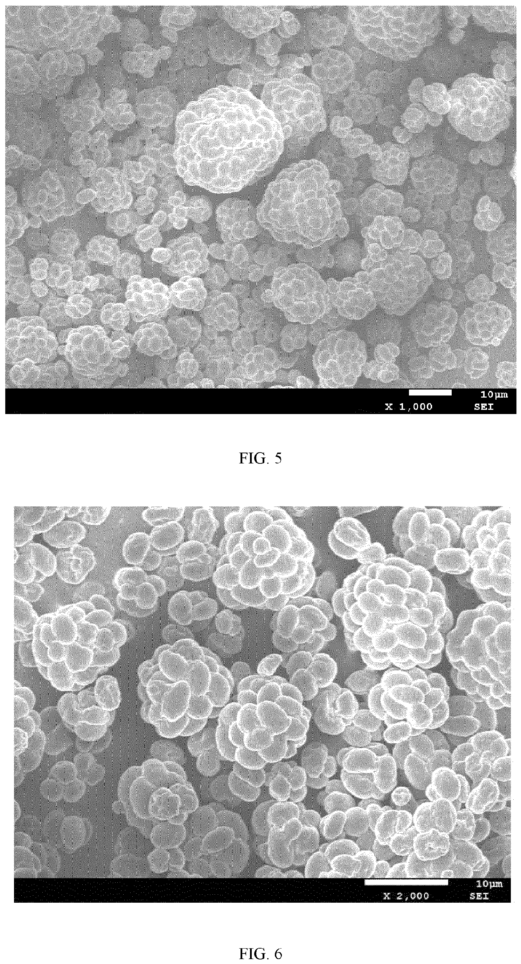

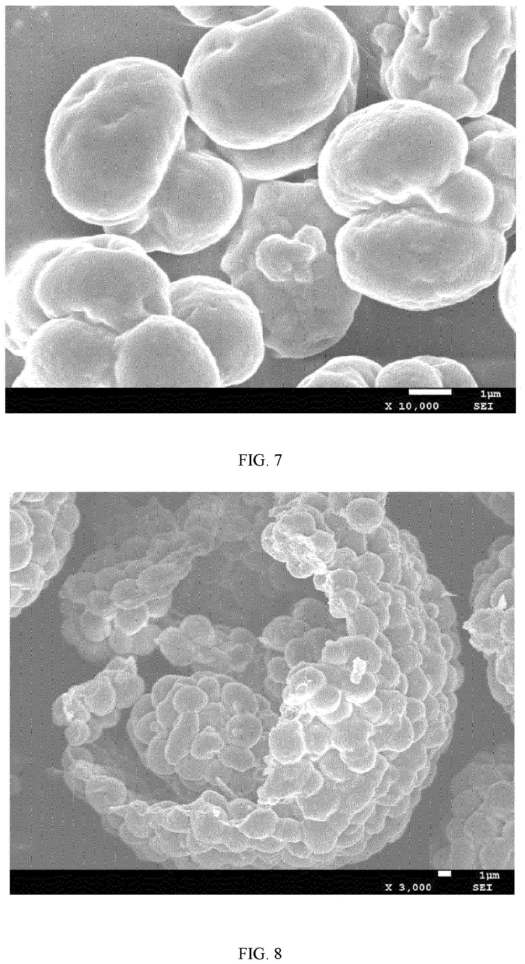

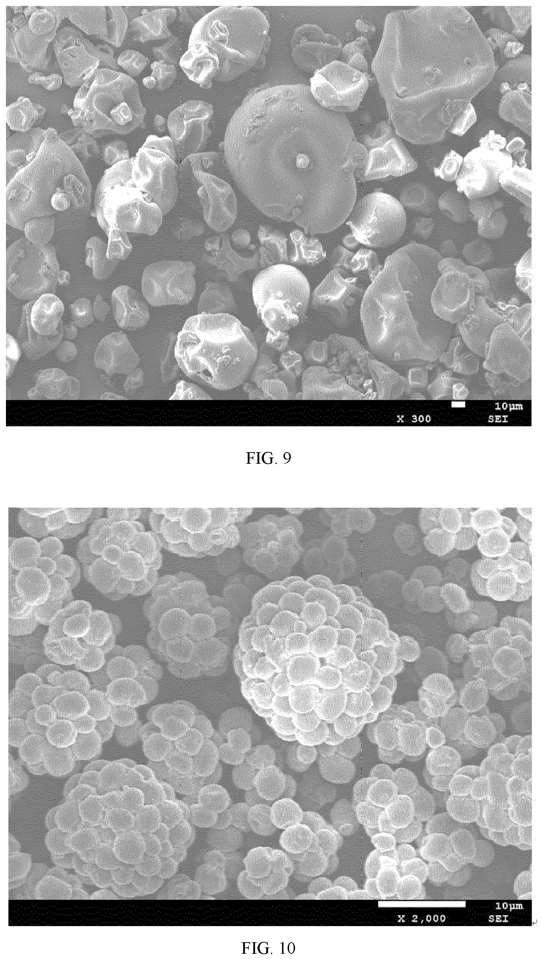

The raw material Saccharomyces cerevisiae powder, the soy protein isolate in Example 1, the yeast protein prepared in Example 1, and the yeast protein prepared in Comparative Example 1 were respectively subjected to a scanning electron microscope SEM test. The scanning electron microscope images of the raw material Saccharomyces cerevisiae powder, the soy protein isolate in Example 1, and the yeast protein prepared in Example 1 are shown in FIGs. 5 to 7, the scanning electron microscope image of the yeast protein prepared in Comparative Example 1 is shown in FIG. 8, the scanning electron microscope image of the soy protein isolate is shown in FIG. 9, and the scanning electron microscope image of the raw material yeast powder in Example 1 is shown in FIG. 10.

It can be seen from FIGs. 5 to 10 that the yeast protein prepared in Example 1 is in the shape of a sphere or an ellipsoid, similar to the structure of the yeast cell powder, and the protein is coated in the cell wall; soy protein isolate is an irregular sphere because the protein is exposed (the composition of soybean protein is mainly globulin); the yeast protein in Comparative Example 1 was homogenized, and although part of the yeast protein was in the shape of an ellipsoid, similar to whole yeast cells, part of the yeast protein was released due to the effect of homogenized wall breaking.

**Experimental Example 1**

The digestion and absorption of yeast protein (YPC) obtained in Example 1 and yeast protein (YPT), soy protein isolate (SPI), and whey protein (WPC) obtained in Comparative Example 1 were compared using an in vitro digestion simulation method as follows:

1) Gastric phase (fed state): digested at 37°C for 2 h while mixing by stirring to lower the pH according to an S-shape curve; normalized pepsin activity was provided by measuring the absorbance at 280 nm of soluble products of hemoglobin (reference protein) after trichloroacetic acid (TCA) digestion; phosphatidylcholine, SHIME nutrient culture medium, sodium chloride, and potassium chloride were added.

2) Small bowel phase (fed state): digested at 37°C for 3 h while mixing by stirring, pH was increased to 7.4; the added pancreatin was an animal pancreatic extract, and the added bile salt was 0.01 mol/L bovine bile extract (bovine bile was closer to human bile than porcine bile for taurine and glycocholate); in addition, bile salts were diluted during digestion to mimic what occurs in the duodenum, jejunum and ileum. Static dialysis using cellulose membranes at the end of digestion (cutoff value = 14 kDa) was used to simulate the absorption process at the level of the small intestine. Digestion conditions (pH profile, digestion time) were optimized to simulate different regions of the gastrointestinal tract in vivo by introducing a small intestinal suspension into the dialysis tube, gradually removing small molecules such as digested amino acids from the matrix of the upper gastrointestinal tract, and all tests were performed 3 times.

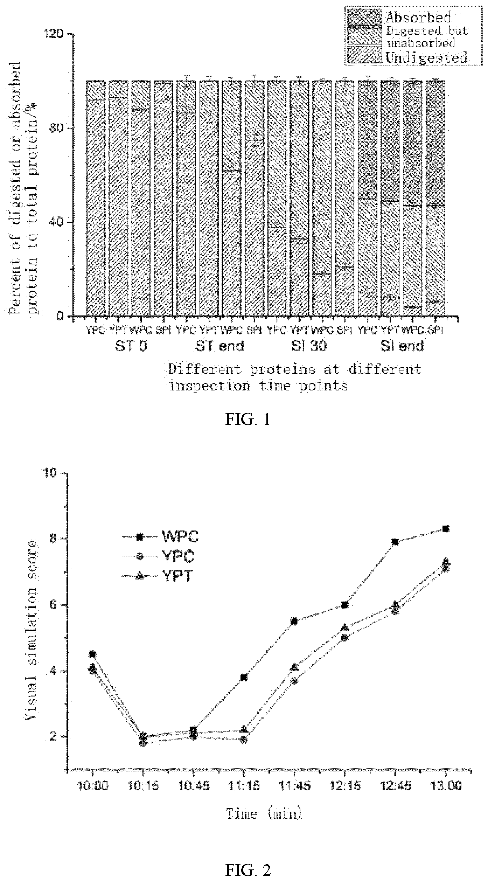

3) The degree of proteolysis was determined by a quantitative non-protein nitrogen (NPN) assay. NPN can be determined by treating the sample with TCA (trichloroacetic acid), which results in precipitation of the protein, followed by filtration. The protein in the filtrate was derived from free amino acids and small peptides formed upon digestion of the protein (and can also be selectively detected with a spectrophotometer). Protein content was determined by total Kjeldahl analysis of the initial and TCA precipitated samples to distinguish between digested and undigested protein components. The following samples were collected at different time points for analysis of protein digestibility or absorptivity: (1) initial product (ST0); (2) end of gastric digestion (ST end); (3) after intestinal digestion for 30 min (SI 30); (4) end of small bowel digestion (SI end); (5) dialyzed mixture suspension at 1 h, 2 h and 3 h (absorbed protein components). The results are shown in FIG. 1.

Digestibility = (X-X1)/X,

Absorptivity = X2/X,

In the formula: X1 is the nitrogen content of TCA precipitated protein, %;

X2 is the total nitrogen content in dialysate, %;

X is the total nitrogen content of a sample collected, %.

4) Determination of background protein content

In this test, a control group was added, and the content of background protein used to simulate different components of the upper gastrointestinal tract was determined by the Kjeldahl method and corrected.

The results are shown in FIG. 1. It can be seen from FIG. 1 that the digestive absorption level of WPC in the distal small intestine was significantly higher than that of YPC, YPT and SPI ($P < 0.01$), but there was no significant difference in the overall digestibility of YPC, YPT and SPI ($P > 0.05$); however, there was no significant difference in the absorptivity of four protein raw materials ($P > 0.05$). However, different proteins have different digestibility in the gastric lumen. Whey protein WPC (38.2%) and soy protein isolate SPI (25.1%) were more obvious than that of yeast protein YPC (13.4%) prepared in Example 1 and yeast protein YPT (15.6%) prepared in Comparative Example 1, the digestion of yeast protein was significantly slower than that of whey protein and soy protein isolate via gastrointestinal digestion, and the digestion of yeast protein YPC prepared in Example 1 was slower than that of yeast protein YPT prepared in Comparative Example 1.

**Experimental Example 2**

In the present invention, the yeast protein (YPC) prepared in Example 1, the yeast protein (YPT) prepared in Comparative Example 1, and the whey protein (WPC) were subjected to a satiety test according to the following method: fifteen volunteers with a mean age of $29\pm3$ years and a mean body mass index of $22.3\pm1.7$ Kg/m$^2$ were recruited for a 2-day test on one of the three diets per day. The subjects did not eat any alcoholic beverages the day before the test, ate a small amount of dinner before 8:00 pm, and fasted until the start of the test. Having three meals per day during the test:

1) Small and standard breakfast consisting of whole flour bread slices, 15 g jam, and milk (150 Kcal). Eating at 7:45 am and taking 10-15 min.

2) The test diets, (5 volunteers received Diet 1, 5 volunteers received Diet 2, and 5 volunteers received Diet 3, as shown in Table 2) were consumed at 10:00 am, taking approximately 15 min.

3) Diets based on tomato paste pasta were consumed at 1:00 pm.

Volunteers scored their stomachs for hunger, feeling hunger, and fullness on a visual analog scale (10 cm) with an interval of 30 min between 10:00 am and 1:00 pm.

When eating at 1:00 pm, volunteers were allowed to eat until they were full, and the amount of pasta and tomato paste ingested was checked and weighed. The subjects note in a notebook the food product consumed for the remainder of the day of the study. The food composition table (1991) using McCance and Widdowson can estimate the amount of Kcal consumed from the amount of various foods consumed during lunch and the rest of the day.

Table 2 Protein Dietary Composition unit g)

| Ingredients | Diet 1 | Diet 2 | Diet 3 |
|---|---|---|---|
| Whey protein | 47.6 | -- | -- |
| Yeast protein in Example 1 | -- | 47.6 | -- |
| Yeast protein in Comparative Example 1 | -- | -- | 47.6 |
| Sucrose | 8.0 | 8.0 | 8.0 |
| Artificial sweetener | 1.6 | 1.6 | 1.6 |

(continued)

| Ingredients | Diet 1 | Diet 2 | Diet 3 |
|---|---|---|---|
| Caramel flavor | 0.8 | 0.8 | 0.8 |
| Caramel colorant | 0.02 | 0.02 | 0.02 |
| Water | 340.0 | 340.0 | 340.0 |

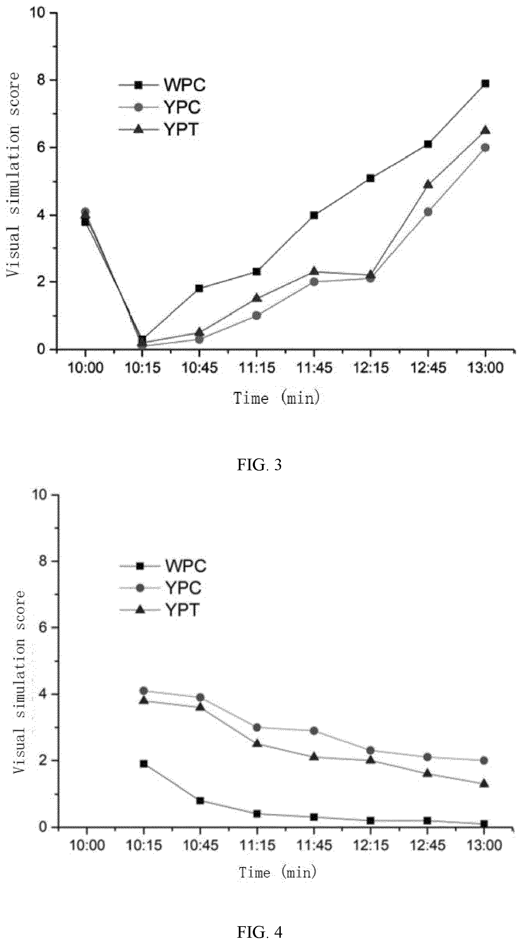

The results of the experiment are shown in FIG. 2, and FIG. 2-4 gives the gastric "feel hunger", "hunger" and "fullness" curves. Example 1 Preparation of yeast protein (YPC) behaves differently than whey protein concentrate (WPC) and comparative example 1 preparation of yeast protein (YPT). Eating a diet based on yeast protein in Example 1 produced hunger and appetite more slowly, and the feeling of stomach fullness lasted longer in the case of yeast protein in Example 1. After the first consumption of whey protein, the yeast protein produced in Comparative Example 1 and the yeast protein produced in Example 1, the average caloric supply at mealtime and for the remainder of the day was compared. As shown in Table 3, from the results in Table 3, when the diet was the yeast protein prepared in Example 1 (i.e. Diet 2), the supply was reduced.

These results show that consumption of the yeast protein prepared in Example 1 is more satiety than whey protein and the yeast protein prepared in Comparative Example 1.

Table 3 Heat supply (in Kcal)

|  | Diet 1 | Diet 2 | Diet 3 |
|---|---|---|---|
| Diet | 1528±650 | 1189±321 | 1320±362 |
| Remainder of the day | 933±273 | 731±262 | 824±325 |
| Diet + remainder of the day | 2461±923 | 1920±583 | 2144±687 |

**Experimental Example 3**

The amino acid compositions of the yeast protein (YPC) prepared in Example 1 and the yeast protein (YPT) prepared in Comparative Example 1 were determined by referring to GB 5009.124-2016 *Determination of Amino acids in Food of National Standard for Food Safety*. The average value was obtained by testing twice. Based on the amino acid score model proposed by FAO/WHO in 1973 and the whole egg protein model proposed by the Institute for Nutrition and Food Safety of the Chinese Center for Disease Control and Prevention, YPC and YPT were compared. The amino acid score (AAS) and chemical score (CS) were calculated according to equations (1) and (2).

AAS = Mass of amino acid in test sample/(Content of same amino acid in FAO/WHO scoring standard mode) (1)
Cs = Content of amino acid in test sample/Content of the same amino acid in egg (2)

Table 4 Amino acid composition, amino acid score, and chemical score of YPC and YPT

|  | Amino acid | FAO/WHO Mode | Egg/ (mg·g⁻¹) | YPC/ (mg·g⁻¹) | YPT/ (mg·g⁻¹) | AAS/ YPC | AAS/YPT | CS/ YPC | CS/ YPT |
|---|---|---|---|---|---|---|---|---|---|
| Essential amino acid | Ile | 40 | 66 | 60.22 | 54.11 | 1.51 | 1.35 | 0.91 | 0.82 |
|  | Leu | 70 | 88 | 9036 | 87.24 | 1.29 | 1.25 | 1.03 | 0.99 |
|  | Lys | 50 | 64 | 96.78 | 85.18 | 1.94 | 1.70 | 1.51 | 1.33 |
|  | Met | 35 | 55 | 25.12 | 24.35 | 0.72 | 0.70 | 0.46 | 0.44 |

(continued)

| | Amino acid | FAO/WHO Mode | Egg/ (mg·g⁻¹) | YPC/ (mg·g⁻¹) | YPT/ (mg·g⁻¹) | AAS/ YPC | AAS/YPT | CS/ YPC | CS/ YPT |
|---|---|---|---|---|---|---|---|---|---|
| | Cys | | | 14.32 | 11.07 | | | | |
| | Phe | 60 | 100 | 49.18 | 49.36 | 0.82 | 0.82 | 0.49 | 0.49 |
| | Tyr | | | 49.24 | 42.59 | | | | |
| | Thr | 40 | 51 | 50.66 | 58.16 | 1.27 | 1.45 | 0.99 | 1.14 |
| | Trp | 10 | 16 | 13.54 | 9.29 | 1.35 | 0.93 | 0.85 | 0.58 |
| | Val | 50 | 73 | 55.25 | 51.43 | 1.11 | 1.03 | 0.76 | 0.70 |
| Non-essential amino acid | Asp | | | 111.17 | 99.45 | | | | |
| | Arg | | | 53.03 | 52.66 | | | | |
| | Ser | | | 52.21 | 44.51 | | | | |
| | Glu | | | 109.91 | 10978 | | | | |
| | Gly | | | 47.37 | 39.42 | | | | |
| | Ala | | | 59.19 | 58.57 | | | | |
| | His | | | 30.02 | 25.69 | | | | |
| | Pro | | | 27.64 | 27.34 | | | | |
| Total amino acid | | | | 995.21 | 930.20 | | | | |

Experimental results are shown in Table 4, it can be seen from Table 4 that the yeast protein YPC prepared in Example 1 and the yeast protein YPT prepared in Comparative Example 1 both contain 18 kinds of amino acids, including 8 kinds of amino acids essential to human body and 2 kinds of semi-essential amino acids, all of which belong to complete proteins. According to the amino acid pattern proposed by FAO/WHO, the total amount of essential amino acids shall be more than 40% of the total amount of amino acids, and the ratio of the total amount of essential amino acids to the total amount of non-essential amino acids shall be more than 0.6. On the basis that the amino acid composition of a protein is close to the ideal pattern of FAO/WHO, a higher ratio indicates a higher nutritional value of the protein. The amino acid composition of YPC and YPT was close to the ideal pattern, and the ratio of essential amino acids to non-essential amino acids reached 1.03, indicating that the nutritional value of the yeast protein prepared in Example 1 was equivalent to that of the yeast protein prepared in Comparative Example 1.

In summary, the outer layer of the yeast protein of the present invention is coated with zymosan, which is in a sphere or ellipsoid shape, the protein content of the yeast protein is more than 70%, and the content of the zymosan is 5-30%; the yeast protein has obviously lower digestibility than that of soy protein isolate and whey protein in the stomach, causes a stronger satiety after being eaten compared with whey protein, has the characteristic of slow digestion, and is a slow-digesting protein. The yeast protein of the present invention also contains 18 amino acids, including eight essential amino acids and two semi-essential amino acids for the human body, and thus is a complete protein due to the full type of amino acids. The yeast protein can be used as the slow-digesting protein in the preparation of a composition which is intended to be enterally administered to mammals so as to regulate the postprandial amino acid level in the blood plasma. The foregoing is illustrative of the preferred embodiments of the present invention and is not to be construed as limiting the invention in any way. Thus, it is intended that the present invention cover the modifications, equivalents, and alternatives falling within the spirit and scope of the invention.

**Claims**

1. A yeast protein, **characterized in that** an outer layer of the yeast protein is coated with zymosan, the yeast protein is in the shape of a sphere or an ellipsoid, and based on taking a dry base weight of the yeast protein as 100%, the yeast protein has a protein content of 70% or more and a zymosan content of 5-30%.

2. The yeast protein of claim 1, **characterized in that** the protein comprises isoleucine, leucine, lysine, methionine, phenylalanine, tyrosine, threonine, cysteine, tryptophan, valine, aspartic acid, arginine, glutamic acid, glycine, histidine, alanine, serine, and proline.

3. The yeast protein of claim 1 or 2, **characterized in that**, by weight of the yeast protein, the content of isoleucine is 60 mg/g or more, the content of leucine is 90 mg/g or more, the content of lysine is 96 mg/g or more, the content of methionine is 25 mg/g or more, the content of phenylalanine is 49 mg/g or more, the content of tyrosine is 49 mg/g or more, the content of threonine is 50 mg/g or more, the content of cysteine is 14 mg/g or more, the content of tryptophan is 13 mg/g or more, the content of valine is 55 mg/g or more, the content of aspartic acid is 111 mg/g or more, the content of arginine is 53 mg/g or more, the content of glutamic acid is 109 mg/g or more, the content of glycine is 47 mg/g or more, the content of histidine is 30 mg/g or more, the content of alanine is 59 mg/g or more, the content of serine is 52 mg/g or more, and the content of proline is 27 mg/g or more.

4. A preparation method for the yeast protein of any one of claims 1 to 3, **characterized by** comprising the following steps:

   (1) adding an enzyme to the yeast for enzymolysis;
   (2) performing thermal extraction on the enzymatic hydrolysate obtained in the step (1), and centrifuging to take a heavy phase to obtain the yeast protein.

5. The preparation method of claim 4, **characterized in that** the enzyme in the step (1) is selected from α-mannanase and/or β-glucanase; preferably, the enzyme is added in an amount of 0.05-0.5 wt%, preferably 0.1-0.3 wt% of the yeast content; further preferably, the enzymolysis temperature is in the range of 40-70°C, preferably 50-60°C, still further preferably, the enzymolysis time is in the range of 8-24 h, preferably 14-18 h.

6. The preparation method of claim 4 or 5, **characterized in that** in the step (1), the yeast is formulated into a solution with a mass concentration of 5%-15%, preferably 8-12%, and preferably the yeast is beer yeast or Saccharomyces cerevisiae.

7. The preparation method of any one of claims 4 to 6, **characterized in that** the thermal extraction pH in the step (2) is 7.0-8.0, preferably 7.5-7.7, preferably the thermal extraction temperature is 70-90°C, preferably 75-80°C, further preferably the thermal extraction time is 1-2 h.

8. The preparation method of any one of claims 4 to 7, **characterized by** further comprising subjecting the yeast protein to a drying step: preparing the yeast protein into a dispersion with a mass concentration of 5-20%, and then drying to obtain, preferably the dispersion has a mass concentration of 10-15%.

9. A yeast protein, **characterized in that** the yeast protein is prepared by the preparation method of any one of claims 4 to 8.

10. A yeast protein composition, **characterized by** comprising at least the yeast protein of any one of claims 1 to 3 or claim 9.

11. The composition of claim 10, **characterized in that** the yeast protein composition further comprises one or a combination of two or more selected from the group consisting of carbohydrates, lipid substances, and free amino acids; preferably, the carbohydrate is one or a combination of two or more selected from the group consisting of corn syrup, maltodextrin, sucrose, oat powder, corn powder, red date flour, red bean flour, Radix Puerariae flour, pumpkin flour, chia seed, and lactose; further preferably, the lipid substance is one or a combination of two or more selected from the group consisting of coconut oil, Brassica oil, corn oil, and soybean lecithin; furthermore preferably, the free amino acid is one or a combination of two or more selected from the group consisting of arginine, L-cystine, L-glutamine, L-glycine, L-histidine, L-isoleucine, L-leucine, L-lysine, L-methionine, L-proline, L-tryptophan, L-tyrosine, and L-valine.

12. Use of the yeast protein of any one of claims 1 to 3 or claim 9 or the composition of claim 10 or 11 in the preparation of a composition intended for enteral administration to a mammal for the modulation of postprandial amino acid level in the blood plasma.

13. The use of claim 12, **characterized in that** the yeast protein or yeast protein composition is used for the preparation of a composition for increasing postprandial protein acquisition.

14. The use of claim 12, **characterized in that** the yeast protein or yeast protein composition is used for the preparation of a composition for reducing the metabolic overload of certain organs and/or certain enzymes.

15. The use of claim 12, **characterized in that** the yeast protein or yeast protein composition is used for the preparation of a composition for limiting daily food intake.

16. The use of claim 12, **characterized in that** the yeast protein or yeast protein composition is used for the preparation of a composition for compensating certain dysfunctions in amino acid metabolism.

17. The use of claim 12, **characterized in that** the yeast protein or yeast protein composition is used for the preparation of a composition for improving tissue regeneration.

18. A food product for patients with renal dysfunction, **characterized by** comprising, in percentage by weight, the following components: the yeast protein of any one of claims 1 to 3 or claim 9 1-10%, carbohydrates 20-30%, and lipid compounds 5-15%, the remainder being water.

19. A meal replacement powder, **characterized by** comprising, in parts by weight, the following components: 5-20 parts of the yeast protein of any one of claims 1 to 3 or claim 9, 5-20 parts of oat powder, 5-25 parts of corn powder, 5-20 parts of red date powder, 5-10 parts of red bean powder, 5-10 parts of Radix Puerariae powder, 5-10 parts of pumpkin powder, and 10-30 parts of chia seed.

20. A food product for patients with phenylketonuria, **characterized by** comprising, in percentage by weight, the following components: the yeast protein of any one of claims 1 to 3 or claim 9 1-5%, free amino acids 1-5%, carbohydrates 10-20% and lipid compounds 1-5%, the remainder being water.

FIG. 1

FIG. 2

FIG. 3

FIG. 4

FIG. 5

FIG. 6

FIG. 7

FIG. 8

FIG. 9

FIG. 10

## INTERNATIONAL SEARCH REPORT

| International application No. |
| --- |
| **PCT/CN2022/133213** |

| A. | CLASSIFICATION OF SUBJECT MATTER |
| --- | --- |

A23J 1/18(2006.01)i; A23L 33/195(2016.01)n; A61K 38/01(2006.01)n; A61P 3/02(2006.01)n

According to International Patent Classification (IPC) or to both national classification and IPC

| B. | FIELDS SEARCHED |
| --- | --- |

Minimum documentation searched (classification system followed by classification symbols)

A23J;A23L;A61K

Documentation searched other than minimum documentation to the extent that such documents are included in the fields searched

Electronic data base consulted during the international search (name of data base and, where practicable, search terms used)

IPCOM; CNTXT; ENTXT; CJFD; ENTXTC; VEN: CNKI; 读秀, DUXIU: 蛋白, 球, 玉米, 赖氨酸, 热提取, 慢消化, 圆形, 球形, 张海波, 酵母多醣, 加热, 龚世禹, 水解, 葡聚糖酶, 酵母葡聚糖, 非消化, 被覆, 包覆, 葛根, 离心, 酶解, 氨基酸, 董运海, 涂覆, 酵母多糖, 亮氨酸, 红枣, 甘露聚糖酶, 熊涛, 不消化, 凝胶, 包埋, 南瓜, 酵母多醣, 燕麦, 水解作用, 难消化, 温度, 包封, 包裹, 下层, 包裹, range[70~90 °C], 陈智仙, 苯丙酮尿, 凝胶, 不易消化, 多醣, 安琪, 酵母蛋白, 重相, 朱银宏, 外层, 酵母, 红豆, 壳, 奇亚籽, 胶, 蛋白; slow digestion, zymosan, Heavy phase, yeast, Yeast protein, enzy+, Heat extraction, digestibility, glucanase, mannanase

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 114027390 A (ANGEL NUTRITECH CO., LTD.) 11 February 2022 (2022-02-11) claims 1-20 | 1-20 |
| X | WO 9705272 A1 (QUEST INTERNATIONAL B.V. et al.) 13 February 1997 (1997-02-13) description, specific embodiment 1, and description, abstract | 1-11 |
| Y | WO 9705272 A1 (QUEST INTERNATIONAL B.V. et al.) 13 February 1997 (1997-02-13) description, specific embodiment 1, and description, abstract | 12–20 |
| Y | CN 1323165 A (SOCIETE DES PRODUITS NESTLE S.A.) 21 November 2001 (2001-11-21) description, p. 2, line 1 to p. 4, line 17, and embodiments 5-13 | 12–20 |
| A | CN 103082081 A (ANGEL YEAST CO., LTD.) 08 May 2013 (2013-05-08) entire document | 1-20 |
| A | CN 109198156 A (ANGEL YEAST CO., LTD.) 15 January 2019 (2019-01-15) entire document | 1-20 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **08 January 2023** | **28 January 2023** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2022/133213**

**C.    DOCUMENTS CONSIDERED TO BE RELEVANT**

| Category* | Citation of document, with indication, where appropriate, of the relevant passages | Relevant to claim No. |
|---|---|---|
| A | US 2021267230 A1 (LESAFFRE ET COMPAGNIE) 02 September 2021 (2021-09-02)<br>       entire document | 1-20 |
| A | WO 2011054255 A1 (ANGELYEAST CO., LTD. et al.) 12 May 2011 (2011-05-12)<br>       entire document | 1-20 |
| A | RU 2011146264 A (УЧРЕЖДЕНИЕ РОССИЙСКОЙ АКАДЕМИИ НАУК ИНСТИТУТ ХИМИИ ТВЕРДОГО ТЕЛА И МЕХАНОХИМИИ СИБИРСКОГО ОТДЕЛЕНИЯ РАН (ИХТТМ СО РАН)) 10 June 2013 (2013-06-10)<br>       entire document | 1-20 |
| A | WO 2020192496 A1 (NANJING AGRICULTURAL UNIVERSITY) 01 October 2020 (2020-10-01)<br>       entire document | 1-20 |
| A | WO 2009132501 A1 (ANGEL YEAST CO., LTD. et al.) 05 November 2009 (2009-11-05)<br>       entire document | 1-20 |
| A | WO 2020200984 A1 (FRIESLANDCAMPINA NEDERLAND B.V.) 08 October 2020 (2020-10-08)<br>       entire document | 1-20 |

Form PCT/ISA/210 (second sheet) (January 2015)

## INTERNATIONAL SEARCH REPORT
### Information on patent family members

International application No.

**PCT/CN2022/133213**

| Patent document cited in search report | | | Publication date (day/month/year) | Patent family member(s) | | | Publication date (day/month/year) |
|---|---|---|---|---|---|---|---|
| CN | 114027390 | A | 11 February 2022 | None | | | |
| WO | 9705272 | A1 | 13 February 1997 | ZA | 965374 | B | 25 December 1997 |
| | | | | ZA | 9605374 | B | 25 December 1997 |
| | | | | AU | 6303796 | A | 26 February 1997 |
| CN | 1323165 | A | 21 November 2001 | AU | 6473999 | A | 08 May 2000 |
| | | | | BR | 9914594 | A | 26 June 2001 |
| | | | | ES | 2188259 | T3 | 16 June 2003 |
| | | | | EP | 1148791 | A1 | 31 October 2001 |
| | | | | US | 6355612 | B1 | 12 March 2002 |
| | | | | WO | 0022937 | A1 | 27 April 2000 |
| | | | | JP | 2002527457 | A | 27 August 2002 |
| | | | | DE | 69904511 | D1 | 23 January 2003 |
| | | | | CN | 1273030 | C | 06 September 2006 |
| | | | | MX | 2001003794 | A1 | 01 January 2002 |
| | | | | MX | 224523 | B | 29 November 2004 |
| | | | | EP | 1148791 | B1 | 11 December 2002 |
| CN | 103082081 | A | 08 May 2013 | CN | 103082081 | B | 25 November 2015 |
| CN | 109198156 | A | 15 January 2019 | CN | 109198156 | B | 08 March 2022 |
| US | 2021267230 | A1 | 02 September 2021 | BR | 112020021753 | A2 | 26 January 2021 |
| | | | | ZA | 202006252 | B | 26 January 2022 |
| | | | | EP | 3784047 | A1 | 03 March 2021 |
| | | | | CA | 3097196 | A1 | 31 October 2019 |
| | | | | RU | 2020138243 | A | 27 May 2022 |
| | | | | SG | 11202010543 Q | A | 27 November 2020 |
| | | | | WO | 2019207111 | A1 | 31 October 2019 |
| | | | | CN | 112105267 | A | 18 December 2020 |
| | | | | KR | 20210002501 | A | 08 January 2021 |
| | | | | JP | 2021521839 | A | 30 August 2021 |
| | | | | FR | 3080521 | A1 | 01 November 2019 |
| | | | | AU | 2019260558 | A1 | 26 November 2020 |
| | | | | IN | 202017049396 | A | 12 February 2021 |
| | | | | VN | 76487 | A | 25 March 2021 |
| | | | | FR | 3080521 | B1 | 09 July 2021 |
| WO | 2011054255 | A1 | 12 May 2011 | EP | 2497833 | A1 | 12 September 2012 |
| | | | | ES | 2676648 | T3 | 23 July 2018 |
| | | | | CL | 2012001181 | A1 | 11 January 2013 |
| | | | | CN | 102051400 | A | 11 May 2011 |
| | | | | CN | 102051400 | B | 15 May 2013 |
| | | | | EP | 2497833 | B1 | 11 April 2018 |
| RU | 2011146264 | A | 10 June 2013 | RU | 2504384 | C2 | 20 January 2014 |
| WO | 2020192496 | A1 | 01 October 2020 | CN | 109912700 | A | 21 June 2019 |
| | | | | CN | 109912700 | B | 09 August 2022 |
| WO | 2009132501 | A1 | 05 November 2009 | JP | 2010533479 | A | 28 October 2010 |
| | | | | ES | 2614286 | T3 | 30 May 2017 |
| | | | | EP | 2272876 | A1 | 12 January 2011 |
| | | | | CN | 101570769 | A | 04 November 2009 |
| | | | | US | 2011045545 | A1 | 24 February 2011 |
| | | | | EP | 2272876 | A4 | 21 March 2012 |
| | | | | CN | 101570769 | B | 19 June 2013 |

Form PCT/ISA/210 (patent family annex) (January 2015)

**INTERNATIONAL SEARCH REPORT**
Information on patent family members

International application No.

**PCT/CN2022/133213**

| Patent document cited in search report | | | Publication date (day/month/year) | Patent family member(s) | | | Publication date (day/month/year) |
|---|---|---|---|---|---|---|---|
| | | | | JP | 5221656 | B2 | 26 June 2013 |
| | | | | US | 8679797 | B2 | 25 March 2014 |
| | | | | EP | 2272876 | B1 | 09 November 2016 |
| | | | | IN | 290422 | B | 15 December 2017 |
| | | | | IN | 201004450 | P2 | 12 August 2011 |
| WO | 2020200984 | A1 | 08 October 2020 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107802001 A
- WO 1997005785 A1
- CN 109198156